# EUROPEAN PATENT APPLICATION

(11) **EP 4 527 928 A1**
(43) Date of publication of application: **26.03.2025**
(21) Application number: 23306581.2
(22) Date of filing: 22.09.2023
(51) Int. Cl.: C12N 15/113, A61K 31/00, A61K 48/00, A61P 25/00, C12Q 1/6883

(54) **POLYNUCLEOTIDES AND USES IN THE TREATMENT OF PRADER-WILLI SYNDROME (PWS) AND /OR PRADER-WILLI LIKE SYNDROME**

(71) Applicant: Université de Lorraine, 54052 Nancy (FR); Centre National de la Recherche Scientifique, 75016 Paris (FR)
(72) Inventor: LABIALLE, Stéphane, 54000 NANCY (FR)
(74) Representative: Novagraaf Technologies

(57) **Abstract**

The invention relates to an isolated polynucleotide comprising the sequence GGXaGGCAXbXcCCXdXeGGAAAGG (SEQ ID NO 1), wherein Xa, Xb, Xc, Xd and Xe are independently T or U, and its use in the treatment of Prader-Willi Syndrome and/or Prader Willi-like syndromes.

The invention also relates to pharmaceutical compositions an isolated polynucleotide comprising the sequence GGXaGGCAXbXcCCXdXeGGAAAGG (SEQ ID NO 1), wherein Xa, Xb, Xc, Xd and Xe are independently T or U and its use in the prevention and/or treatment of Prader-Willi Syndrome and/or Prader Willi-like syndromes.

The present invention finds applications in the therapeutic and diagnostic medical technical fields.

## Description

### FIELD

The invention relates to an isolated polynucleotide comprising the sequence GGXaGGCAXbXcCCXdXeGGAAAGG (SEQ ID NO 1), wherein Xa, Xb, Xc, Xd and Xe are independently T or U.

The invention also relates to an isolated polynucleotide for use in the treatment of Prader-Willi Syndrome and Prader Willi-like syndromes.

The invention also relates to pharmaceutical compositions an isolated polynucleotide comprising the sequence GGXaGGCAXbXcCCXdXeGGAAAGG (SEQ ID NO 1), wherein Xa, Xb, Xc, Xd and Xe are independently T or U and to pharmaceutical compositions an isolated polynucleotide comprising the sequence GGXaGGCAXbXcCCXdXeGGAAAGG (SEQ ID NO 1), wherein Xa, Xb, Xc, Xd and Xe are independently T or U for use in the prevention and/or treatment of Prader-Willi Syndrome and Prader Willi-like syndromes.

The present invention finds applications in the therapeutic and diagnostic medical technical fields.

In the description below, the references between square brackets ([ ]) refer to the list of references presented at the end of the text.

### BACKGROUND

Prader Willi syndrome (PWS) is a rare neurodevelopmental disorder that affects approximately 1/15,000 births. Symptoms are classically characterized by hypotonia and growth retardation in early neonatal life. Later on, developmental delay is observed in the child, including short stature, hyperphagia which can lead to morbid obesity if not controlled, as well as cognitive and behavioral disorders (intellectual deficit, obsessivecompulsive behaviors, sleep abnormalities). The syndrome is caused by a deficiency in the paternal expression of genes at the 15q11-q13 locus, in particular a series of tandem repeat genes that allow the expression of small non-coding RNAs called snord116 whose functions are currently unknown (Sahoo et al. 2008 [13]; de Smith et al. 2009 [4]; Duker et al. 2010 [5]; Tan et al. 2020 [16]).

Normally, SNORD116 genes are expressed only from the paternal chromosome of individuals, the copies carried by the maternal chromosome are naturally epigenetically extinguished (parental genomic imprinting phenomenon). In the majority of cases, PWS patients suffer from a loss of expression of SNORD116 genes due to a genetic deletion or epigenetic alteration of the paternal chromosome only. A strategy would be to allow re-expression of genes carried by the maternal chromosome and work has been done in this direction (Cruvinel et al. 2014 [3]; Kim et al. 2017 [10]). However; problems with this epigenetic correction appear, in particular (i) the targeting of general epigenetic lead to genome-wide effects and (ii) a minor reactivation of expression from the maternal chromosome that obviates the hopes for efficacy of the strategy corresponding to 10% of normal expression in the study where SNORD116 were directly assessed (Kim et al. 2017 [10]).

There is therefore a real need to find a method and/or a compound which allows more efficient treatment and/or effective treatment of Prader Willi syndrome (PWS). In particular there is a real need to find new strategies, i.e. new targets/pathways, in the treatment of Prader Willi syndrome (PWS).

In a recent work, molecular RNA targets of the snord116 snoRNAs has been identified (Baldini et al. 2022 [1]). The RNA targets are encoded by the DGKK, NLGN3 and RSBN1L genes that are aberrantly expressed as described in the literature when snord116 expression is artificially decreased in the cellular models as well as, as described in the literature, in mouse models (Zahova 2021 [19]; Knott 2022 [18]) and in induced pluripotent cells from PWS patient biopsies (Burnett 2017 [17]). The DGKK gene that is overexpressed in PWS patients is also a key player in Fragile X syndrome (FXS) while these syndromes share several clinical manifestations. However, in Fragile X syndrome (FXS the DGKK gene is underexpressed (Tabet et al. 2016 [15]). The DGKK gene is targeted for the treatment of Fragile X syndrome (FXS), in particular treatments to increase the expression of the DGKK gene is studied for the treatment of Fragile X syndrome (FXS). However, there is no study nor treatment associated with the Prader Willi syndrome (PWS).

There is therefore a real need to find a method and/or a compound which allows an efficient treatment and/or effective treatment of Prader Willi syndrome (PWS). In particular there is a real need to find new strategies, i.e. new targets/pathways, in the treatment of Prader Willi s yndrome (PWS).

### Description of the invention

The present invention meets these needs and overcomes the abovementioned drawbacks of the prior art by providing an isolated polynucleotide comprising the sequence GGXaGGCAXbXcCCXdXeGGAAAGG (SEQ ID NO 1), wherein Xa, Xb, Xc, Xd and Xe are independently T or U.

The inventors have surprisingly demonstrated that the products of expression of SNORD116 genes interact with target mRNAs produced by the DGKK, NLGN3 and RSBN1L genes, and downregulates their expression.

The inventors have demonstrated that the polynucleotide comprising the sequence GGXaGGCAXbXcCCXdXeGGAAAGG (SEQ ID NO 1), wherein Xa, Xb, Xc, Xd and Xe are independently T or U according to the invention allows to interact with target mRNAs produced by the DGKK, NLGN3 and RSBN1L genes. In particular, the inventor has demonstrated that the polynucleotide of the present invention allows reproduce the effect of snord116 snoRNAs with target mRNAs produced by the DGKK, NLGN3 and RSBN1L genes and thus to regulate the expression of these genes. In addition, the inventors have demonstrated that the polynucleotide of the invention allow to restore the expression level of the DGKK gene that is robustly expressed in undifferentiated induced pluripotent stem cells from PWS patients, i.e. to have a normal expression level of the DGKK gene, allowing therefore the treatment of PWS and/or Prader Willy-like syndrome.

### Definitions

To facilitate an understanding of the present invention, a number of terms and phrases are defined below:
As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof" "a mixture thereof" and such like following a listing, the use of "and/or" as part of a listing, a listing in a table, the use of "etc." as part of a listing, the phrase "such as," and/or a listing within brackets with "e.g.," or i.e., refers to any combination (e.g., any subset) of a set of listed components, and combinations and/or mixtures of related species and/or embodiments described herein though not directly placed in such a listing are also contemplated. Such related and/or like genera(s), sub-genera(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."

As used herein, the term "and/or" means any one of the items, any combination of the items, or all of the items with which this term is associated.

As used herein, the term "about" refers to a variation of ±5-10% of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., weight percents, proximate to the recited range that are equivalent in terms of the functionality of the individual ingredient, the composition, or the embodiment.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range (e.g., weight percents) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to," "at least," "greater than," "less than," "more than," "or more," and the like, include the number recited and such terms refer to ranges that can be subsequently broken down into subranges as discussed above. In the same manner, all ratios recited herein also include all sub-ratios falling within the broader ratio.

An "effective amount" refers to an amount effective to treat a disease, disorder, and/or condition, or to bring about a recited effect. For example, an amount effective can be an amount effective to reduce the progression or severity of the condition or symptoms being treated. Determination of a therapeutically effective amount is well within the capacity of persons skilled in the art. The term "effective amount" is intended to include an amount of an isolated polynucleotide described herein, e.g., that is effective to treat or prevent a disease or disorder, or to treat the symptoms of the disease or disorder, in a host. Thus, an "effective amount" generally means an amount that provides the desired effect.

The terms "treating", "treat" and "treatment" include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, therapeutic, and/or prophylactic administration, as appropriate.

In the present invention, the isolated polynucleotide according to the invention may comprise a nucleic acid sequence having at least 65%, 67% 69% 70%, 75%, 80%, 85%, 90%, 91%, 92%, 93%, 94%, 95%, 96%, 97%, 98%, 99%, 99.5% or 100% sequence identity with the sequence SEQ ID NO 1.

In the present invention, the isolated polynucleotide according to the invention may have at least 65%, 67% 69% 70%, 75%, 80%, 85%, 90%, 91%, 92%, 93%, 94%, 95%, 96%, 97%, 98%, 99%, 99.5% or 100% sequence identity with sequence GGTGGCATTCCTTGGAAAGG (SEQ ID NO 2).

In the present invention, the isolated polynucleotide according to the invention may have at least 65%, 67% 69% 70%, 75%, 80%, 85%, 90%, 91%, 92%, 93%, 94%, 95%, 96%, 97%, 98%, 99%, 99.5% or 100% sequence identity with sequence GGUGGCAUUCCUUGGAAAGG (SEQ ID NO 3).

In the present invention, the isolated polynucleotide according to the invention may be selected from the group comprising the isolated polynucleotide is of sequence GGTGGCATTCCTTGGAAAGG (SEQ ID NO 2) or GGUGGCAUUCCUUGGAAAGG (SEQ ID NO 3).

In the present invention, the isolated polynucleotide according to the invention may be the isolated polynucleotide is of sequence GGTGGCATTCCTTGGAAAGG (SEQ ID NO 2).

In the present, the term "isolated" and its grammatical equivalents as used herein refer to the removal of a nucleic acid from its natural environment.

The percentage of sequence identity may be determined by any method known to one skilled in the art. It may be determined for example by use of BLASTP and BLASTN, for example using default parameters.

In the present, the terms "identical" and its grammatical equivalents as used herein and/or "sequence identity" in the context of two nucleic acid sequences or of two amino acid sequences of polypeptides refers to the residues in a sequence which are the same when aligned for maximum correspondence over a sequence length of at least 20 contiguous nucleic acid or amino acids. It may be for example two sequences which are the same when aligned for maximum correspondence over a sequence length of at least about 50 at least about 200 contiguous nucleic acid or amino acids. The identity of the sequence may be determined by comparing a sequence to a reference sequence of the same number of contiguous positions after the two sequences are aligned optimally.

The determination of the identity and/or the comparison of a sequence to a reference sequence may be carried out with any methods and/or process adapted known to one skilled in the art. It may be for example a method of alignment of sequences for comparison, for example a method of alignment of sequences using the local homology algorithm of Smith and Waterman, Adv. Appl. Math., 2:482 (1981) [23]; using the alignment algorithm of Needleman and Wunsch, J. Mol. Biol., 48:443 (1970) [24]; using a search for similarity method of Pearson and Lipman, Proc. Nat. Acad. Sci U.S.A., 85:2444 (1988) [25]; using a computerized implementations of these algorithms, for example CLUSTAL in the PC/Gene program by Intelligences, Mountain View Calif, GAP, BESTFIT, BLAST, FASTA, and TFASTA in the Wisconsin Genetics Software Package, Genetics Computer Group (GCG), 575 Science Dr., Madison, Wis., U.S.A.) Alignment is also often performed by inspection and manual alignment.

In the present, the term "purified" and its grammatical equivalents as used herein refer to a molecule or composition, whether removed from nature (including genomic DNA and mRNA) or synthesized (including cDNA) and/or amplified under laboratory conditions, that has been increased in purity, wherein "purity" is a relative term, not "absolute purity." It is to be understood, however, that nucleic acids and proteins can be formulated with diluents or adjuvants and still for practical purposes be isolated. For example, nucleic acids may be mixed with an acceptable carrier or diluent when used for introduction into cells.

In the present, the term "substantially purified" and its grammatical equivalents as used herein refer to a nucleic acid sequence, polypeptide, protein or other compound which is essentially free, i.e., is more than about 50% free of, more than about 70% free of, more than about 90% free of, the polynucleotides, proteins, polypeptides and other molecules that the nucleic acid, polypeptide, protein or other compound is naturally associated with.

In the present, "polynucleotide(s)", "oligonucleotide(s)", "nucleic acid(s)", "nucleotide(s)", "polynucleic acid(s)", or any grammatical equivalent as used herein refers to a polymeric form of nucleotides or nucleic acids of any length, either ribonucleotides or deoxyribonucleotides. This term refers only to the primary structure of the molecule. Thus, this term includes double and single stranded DNA, triplex DNA, as well as double and single stranded RNA. It also includes modified, for example, by methylation and/or by capping, and unmodified forms of the polynucleotide. The term is also meant to include molecules that include non-naturally occurring or synthetic nucleotides as well as nucleotide analogs. The nucleic acid sequences and vectors disclosed or contemplated herein can be introduced into a cell by, for example, transfection, transformation, or transduction.

In the present "transfection," "transformation," or "transduction" refer to the introduction of one or more exogenous polynucleotides into a host cell by using physical or chemical methods. It may be any transfection and/or transformation and/or transduction adapted method known to one skilled in the art. It may be for example, calcium phosphate DNA co-precipitation (see, e.g., Murray E. J. (ed.), Methods in Molecular Biology, Vol. 7, Gene Transfer and Expression Protocols, Humana Press (1991) [26]); DEAE- dextran; electroporation; cationic liposome-mediated transfection; tungsten particle-facilitated microparticle bombardment (Johnston, Nature, 346: 776-777 (1990) [27]); and strontium phosphate DNA co-precipitation (Brash et al., Mol. Cell Biol., 7: 2031-2034 (1987) [28]). Phage, viral, or non- viral vectors can be introduced into host cells, after growth of infectious particles in suitable packaging cells, many of which are commercially available. In some embodiments, lipofection, nucleofection, or temporary membrane disruption (e.g., electroporation or deformation) can be used to introduce one or more exogenous polynucleotides into the host cell.

In the present, the nucleic acid may be produced and/or obtained by any method adapted known from one skilled in the art. For example, the nucleic acid may be synthesized using the BigDye^{™} Terminator v3.1 Cycle Sequencing Kit (thermofisher, 4337454) or any device known from one skilled in the art commercially available, for example commercialized by Eurofin and/or Qiagen.

The isolated polynucleotide according to the present invention may arbitrarily comprise at least one modification, for example a base analog, another artificial base, another modified base, or the like.

According to the invention, the isolated polynucleotide of the present invention may comprise at least one locked nucleic acids (LNA). The locked nucleic acids may be any locked nucleic acids known from one skilled in the art. The ribose moiety of an LNA nucleotide is modified from a typical ribose ring structure by a methylene bridge that connects the 2' oxygen atom and the 4' carbon atom, and which locks the ribose in the 3'endo conformation. Such LNAs can comprise any natural purine or pyrimidine base or non-natural bases (e.g., inosine, chemically modified bases, etc.).

According to the present invention, isolated polynucleotide of the present invention may comprise at least one sugar residue been modified or modified sugar residue. For example, the site to be modified in a sugar residue, one having the oxygen atom at the 2'-position, 3'-position and/or 4- position of the sugar residue may be replaced with another atom and/or substituted any adapted chemical group known to one skilled in the art. For example, the at least one modified sugar may comprise a fluorination, O-alkylation (e.g., O-methylation, O-ethylation), O-arylation, S-alkylation (e.g., S-methylation, S-ethylation), S-arylation, and amination. The modification of the sugar may be carried out by any method know to one skilled in the art. For example, the sugar may be modified according to any of the method as disclosed in Sproat et al., (1991) Nucle. Acid. Res. 19, 733-738 [29]; Cotten et al., (1991) Nucl. Acid. Res. 19, 2629-2635 [30]; Hobbs et al., (1973) Biochemistry 12, 5138-5145 [31].

Each of the nucleotides contained in the isolated polynucleotide of the present invention, whether identical or different, may be a nucleotide comprising a hydroxyl group at the 2' position of ribose, for example ribose of pyrimidine nucleotide, ribose of purine nucleotide, i.e., an unsubstituted nucleotide or a nucleotide substituted by any atom or group at the 2' position of ribose.

According to the invention, the isolated polynucleotide of the present invention may comprise at least one substituted ribose at the 2' position by a hydrogen atom, a fluorine atom, an -O-alkyl group, for example -O-Me group, for example -O-2-methoxyethyl group, an -O-acyl group, for example -O-COMe group), or an amino group, for example -NH₂ group. For example the purine nucleotide may be modified to be for example 2'-O-2-methoxyethyl purine.

According to the invention, the isolated polynucleotide of the present invention may comprise at least one modified pyrimidine nucleotide.

According to the invention the modified pyrimidine may be any modified pyrimidine known to one skilled in the art. It may be for example a chemically modified pyrimidine and/or a substituted pyrimidine, for example at the position 2'.

According to the present invention the isolated polynucleotide may comprise at least one, two, three, four of the hydroxy groups at the 2'-positions of respective pyrimidine nucleotides contained in the isolated polynucleotide, whether identical or not, be substituted by any adapted chemical group known to one skilled in the art. It may be for example an atom or a group selected from the group consisting of a hydrogen atom, a fluorine atom, an amino group and a methoxy group.

According to the present invention each of the hydroxy groups at the 2'-positions of respective pyrimidine nucleotides contained in the isolated polynucleotide, whether identical or not, may be substituted by any adapted chemical group known to one skilled in the art. It may be for example an atom or a group selected from the group consisting of a hydrogen atom, a fluorine atom, an amino group and a methoxy group. For example, the pyrimidine nucleotide may be modified to be a 2'-OMe, 2'-fluoro or 2'-O-2-methoxyethyl pyrimidine.

Advantageously, the modification and/or substitution at the 2'-position of the pyrimidine may increase the biostability of the isolated polynucleotide.

According to the invention, the isolated polynucleotide of the present invention may comprise at least one modified purine nucleotide.

According to the invention the modified purine may be any modified purine known to one skilled in the art. It may be for example a chemically modified purine and/or a substituted purine, for example at the position 2'.

According to the present invention the isolated polynucleotide may comprise at least one, two, three, four of the hydroxy groups at the 2'-positions of respective purine nucleotides contained in the isolated polynucleotide, whether identical or not, be substituted by any adapted chemical group known to one skilled in the art. It may be for example an atom or a group selected from the group consisting of a hydrogen atom, a fluorine atom, an amino group and a methoxy group.

According to the present invention each of the hydroxy groups at the 2'-positions of respective purine nucleotides contained in the isolated polynucleotide, whether identical or not, may be substituted by any adapted chemical group known to one skilled in the art. It may be for example an atom or a group selected from the group consisting of a hydrogen atom, a fluorine atom, an amino group and a methoxy group. For example, the purine nucleotide may be modified to be a 2'-OMe, 2'-fluoro or 2'-O-2-methoxyethyl purines.

Advantageously, the modification and/or substitution of the purine at the 2'-position may increase the biostability of the isolated polynucleotide.

The phosphate group contained in the isolated polynucleotide of the present invention may be modified and/or substituted. The modification may be any adapted modification known to one skilled in the art. For example, the P(O)O group may be substituted with P(O)S (thioate), P(S)S (dithioate), P(O)NR₂ (amidate), P(O)CH₃, P(O)BH₃, P(O)R, R(O)OR', CO or CH₂ (formacetal) or 3'-amine (-NH-CH₂-CH₂-), wherein each unit of R or R' is independently H or a substituted or unsubstituted alkyl, for example methyl, ethyl. Preferably, the P(O)O group may be substituted with P(O)S (thioate).

Advantageously, the modification and/or substitution of the phosphate group may increase the biostability of the isolated polynucleotide and/or the resistance of the isolated polynucleotide to nuclease and/or hydrolysis.

According to the invention, the isolated polynucleotide may comprise modification at its 3' and 5'. It may be any modification known to one skilled in the art adapted to be on the 3' and/or 5' end of a nucleotide. It may be for example adding to an end a polyethyleneglycol, amino acid, peptide, inverted dT, nucleic acid, nucleosides, Myristoyl, Lithocolic-oleyl, Docosanyl, Lauroyl, Stearoyl, Palmitoyl, Oleoyl, Linoleoyl, other lipids, steroids, cholesterol, caffeine, vitamins, pigments, fluorescent substances. It may be for example any modification disclosed in U.S. Pat. Nos. 5,660,985 [32] and/or 5,756,703 [33].

The inventor has surprisingly demonstrated and is the first to demonstrate that the isolated polynucleotide of the invention allows to regulate and restore a wild-type expression of genes dysregulated in patient suffering from Prader Willy Syndrome (PWS).

In addition, the inventor has also surprisingly demonstrated and is the first to demonstrate that the isolated polynucleotide of the invention allows to regulate the expression of genes involved in PWS to a wild-type expression allowing therefore the treatment of PWS and/or Prader Willy-like syndrome.

Accordingly, another object of the present invention is an isolated polynucleotide of the invention for its use as medicament.

The isolated polynucleotide is as defined above

The medicament may use for the treatment of a disease selected from the group comprising Prader Willy Syndrome and Prader Willy-like syndrome.

Accordingly, an object of the present invention is an isolated polynucleotide of the invention for use in the treatment of Prader Willy Syndrome and/or Prader Willy-like syndrome.

In the present Prader Willi syndrome (PWS) means is a rare neurodevelopmental disorder that affects approximately 1/15,000 births caused by a deficiency in the paternal expression of genes at the 15q11-q13 locus.

In the present Prader Willy-like syndrome (PWL) means any disease known from one skilled in the art harboring clinical characteristics similar to PWS comprising developmental delay, intellectual disability, speech problems, overweight/obesity, hypotonia and psycho-behavioral problems without a typical genetic aberration of the 15q11-q13 region. It may be, for example a disease involving a maternal uniparental disomy 14, for example Temple syndrome, at least one truncating mutations in the MAGEL2 gene, for example a Schaaf-Yang syndrome, a 1P36 deletion, a 2p deletion, a 6q deletion, a 6q duplication, a 15q duplication, a 19p deletion, a Xq duplication or fragile X syndrome. It may be a syndrome and/or a disease caused by a maternal uniparental disomy 14, at least one truncating mutation in the MAGEL2 gene, an 1P36 deletion, an 2p deletion, an 6q deletion, an 6q duplication, an 15q duplication, an 19p deletion, a Xq duplication. It may be a syndrome and/or a disease as disclosed in Rocha et al. 2014 [12] or in Juriaans et al. 2021 [7]. It may be a syndrome selected from the group comprising Temple syndrome, Schaaf-Yang syndrome, fragile X syndrome. PWL may be a disease comprising any dysregulation of the expression of snord116 genes and/or a dysregulation of the expression of at least one gene selected from the group comprising Dgkk, Nlgn3, Rsbn1L and Nhlh2.

The medicament may be in any form that can be administered to a human or an animal. It may for example be a pharmaceutical composition.

The administration of the medicament may be carried out by any way known to one skilled in the art. It may, for example, be carried out directly, i.e. pure or substantially pure, or after mixing of the isolated polynucleotide of the invention with a pharmaceutically acceptable carrier and/or medium. According to the present invention, the medicament may be an injectable solution, a medicament for oral administration, for example selected from the group comprising a liquid formulation, a multiparticle system, an orodispersible dosage form. According to the present invention, the medicament may be a medicament for oral administration selected from the group comprising a liquid formulation, an oral effervescent dosage form, an oral powder, a multiparticle system, an orodispersible dosage form.

According to the invention, the isolated polynucleotide according to the invention may be used as a medicament, for example, in a pharmaceutical composition.

Another object of the present invention is a pharmaceutical composition comprising the isolated polynucleotide according to the invention.

Another object of the present invention is a pharmaceutical composition comprising the isolated polynucleotide of the invention for use in the prevention and/or treatment of disease.

The isolated polynucleotide of the invention is as defined above.

The disease may be selected from the group comprising Prader Willy Syndrome and Prader Willy-like syndrome.

According to the present invention, the pharmaceutical composition may comprise any pharmaceutically acceptable and/or therapeutically effective amount of the isolated polynucleotide of the invention.

The pharmaceutical composition may be in any form that can be administered to a human or an animal. The person skilled in the art clearly understands that the term "form" as used herein refers to the pharmaceutical formulation of the medicament for its practical use. For example, the medicament may be in a form selected from the group comprising an injectable form, an oral suspension, a pellet, a powder, granules or topical form (e.g. cream, lotion, collyrium).

The pharmaceutical composition may comprise a pharmaceutically acceptable carrier.

The pharmaceutically acceptable carrier may be any known pharmaceutical support used for the administration of an isolated polynucleotide to a human or animal, depending on the subject to be treated. It may be for example a pharmaceutically acceptable carrier selected from the group comprising, excipients such as sucrose, starch, mannitol, sorbitol, lactose, glucose, cellulose, talc, calcium phosphate, and calcium carbonate; binders such as cellulose, methylcellulose, hydroxylpropylcellulose, polypropylpyrrolidone, gelatin, gum arabic, polyethylene glycol, sucrose, and starch; disintegrants such as starch, carboxymethylcellulose, hydroxylpropylstarch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizinammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like.

According to the invention, the pharmaceutical composition may be administrated by any adapted route of administration known to one skilled in the art. For example, the pharmaceutical composition may be administrated by oral administration, by parenteral administration, by intrathecal administration.

For example, for parenteral administration aqueous and non-aqueous isotonic sterile injectable liquids may be used, which may comprise an antioxidant, a buffer solution, a bacteriostatic agent, an isotonizing agent and the like. Aqueous and non-aqueous sterile suspensions can also be mentioned, which may comprise a suspending agent, a solubilizer, a thickener, a stabilizer, an antiseptic and the like. The preparation may be included in a container such as an ampoule or a vial in a unit dosage volume or in several divided doses.

For example, for intrathecal administration aqueous and non-aqueous isotonic sterile injectable liquids may be used.

The pharmaceutical form or method of administering a pharmaceutical composition may be selected with regard to the human or animal subject to be treated. For example, for a child, for example from 1 to 17 years old, or a baby, for example under 1 year old, a syrup or an injection may be preferred. Administration may for example be carried out with a weight graduated pipette, a syringe. For example, for an adult over 17 years old, an injection may be preferred. Administration may be carried out with an intravenous weight graduated syringe.

In the present, the isolated polynucleotide of the invention may be used in a combination therapy, for example with a therapeutic agent.

"Combination therapy" (or "co-therapy") includes the administration of the isolated polynucleotide of the invention and/or pharmaceutical composition of the invention, and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected).

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

Sequential or substantially simultaneous administration of each therapeutic agent can be carried out by any appropriate route including, but not limited to, topical routes, oral routes, intravenous routes, intramuscular routes, intrathecal route, and direct absorption through mucous membrane tissues. The therapeutic agents can be administered by the same route or by different routes. For example, a first therapeutic agent of the combination selected may be administered by injection while the other therapeutic agents of the combination may be administered topically.

A "therapeutic agent" is any compound known in the art that is used in the detection, diagnosis, or treatment of a condition or disease. Such compounds may be naturally-occurring, modified, or synthetic. Non-limiting examples of therapeutic agents may include drugs, therapeutic compounds, genetic materials, metals (such as radioactive isotopes), proteins, peptides, carbohydrates, lipids, steroids, nucleic acid based materials, or derivatives, analogues, or combinations thereof in their native form or derivatized with hydrophobic or charged moieties to enhance incorporation or adsorption into a cell. Non-limiting examples of therapeutic agents may include immune-related agents, thyroid agents, respiratory products, antineoplastic agents, anti-helmintics, anti-malarials, mitotic inhibitors, hormones, anti-protozoans, anti-tuberculars, cardiovascular products, blood products, biological response modifiers, anti-fungal agents, vitamins, peptides, anti-allergic agents, anti-coagulation agents, circulatory drugs, metabolic potentiators, anti-virals, anti-anginals, antibiotics, antiinflammatories, anti-rheumatics, narcotics, cardiac glycosides, neuromuscular blockers, sedatives, local anesthetics, general anesthetics, or radioactive atoms or ions.

Other advantages may still be apparent to those skilled in the art by reading the examples below, illustrated by the accompanying figures, given by way of illustration.

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

### Brief description of the drawing

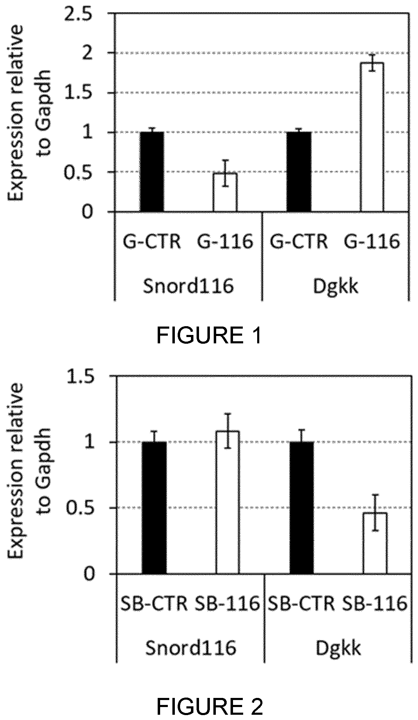

- Figure 1 is a bar chart representing the expression level of snord116 and Dgkk mRNAs relative to Gapdh control mRNA in human cervix carcinoma HeLa S3 cells. Black histograms represent the expression level in cells transfected with a control oligonucleotide (G-CTR) of sequence GCTCCCTTCAATCCAA (SEQ ID NO 10), white histograms represent the expression level in cells transfected with an oligonucleotide of sequence TCACTCATTTTGTTCA (SEQ ID NO 11) directed against endogenous snord116 (G-116) generating their knockdown.
- Figure 2 is a bar chart representing the expression level of snord116 and Dgkk mRNAs relative to Gapdh control mRNA in human cervix carcinoma HeLa S3 cells. Black histograms represent the expression level in cells transfected with a control oligonucleotide (SB-CTR) of sequence TAACACGTCTATACGCCCA (SEQ ID NO 12), white histograms represent the expression level in cells transfected with a oligonucleotide according to the invention of sequence GGTGGCATTCCTTGGAAAGG (SEQ ID NO 2) which allows mimicking the effect of snord116 on the target mRNA (SB-116).

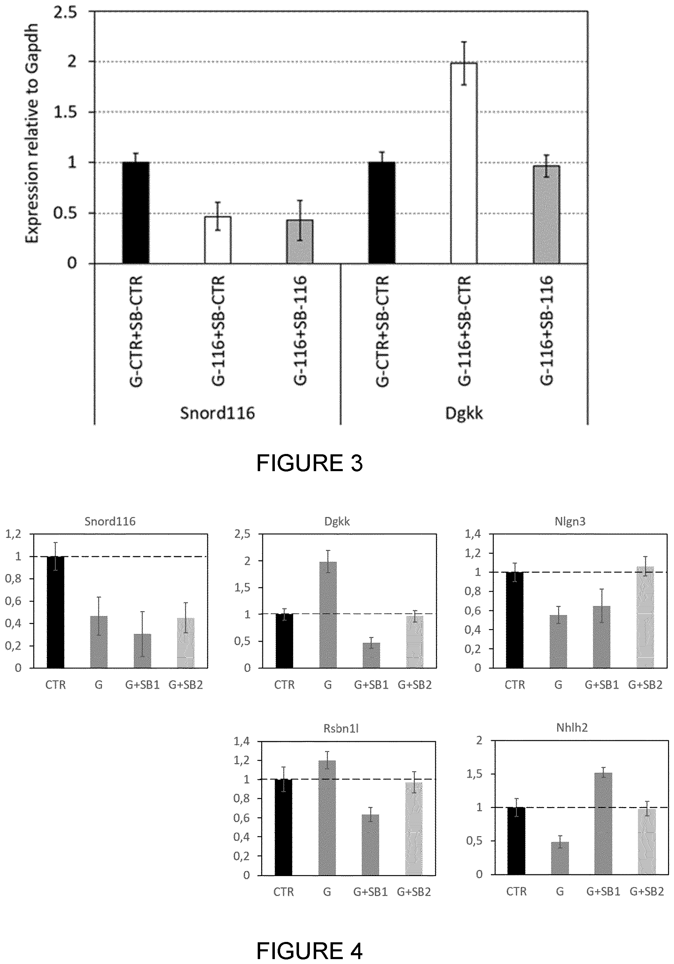

- Figure 3 is a bar chart representing the expression level of snord116 and Dgkk mRNAs relative to Gapdh control mRNA in mouse neuroblastoma Neuro2A cells. Black histograms represent the expression level in cells transfected with control oligonucleotides (G-CTR of SEQ ID NO 10 and SB-CTR of SEQ ID NO 12), white histograms represent the expression level in cells transfected with an oligonucleotide of SEQ ID NO 11 directed against endogenous snord116 allowing their knockdown (G-116) and with a control oligonucleotide of SEQ ID NO 12 (SB-CTR), the shaded histograms the expression level in cells transfected the treatment with the G-116 oligonucleotide of SEQ ID NO 11 directed against endogenous snord116 allowing their knockdown and with a oligonucleotide according to the invention of SEQ ID NO 2 reproducing the effect of snord116 on the target mRNA (SB-116).
- Figure 4 is a bar chart representing the expression level of snord116, Dgkk, Nlgn3, Rsbn1L and Nhlh2 mRNAs relative to Gapdh control mRNA CTR represent the expression level in cells transfected with control oligonucleotides (CTR of SEQ ID NO 10 and SB-CTR (CTR) of SEQ ID NO 12), G represents the expression level in cells transfected with an oligonucleotide of SEQ ID NO 11 and control oligonucleotide of SEQ ID NO 12, G+SB1 represents the expression level in cells transfected with an oligonucleotide of SEQ ID NO 11 (G) and with an oligonucleotide of sequence AACATTCCTTGGAAAA (SB1 of SEQ ID NO 13), G+SB2 represent the expression level in cells transfected with an oligonucleotide of SEQ ID NO 11 (G) and with an oligonucleotide of SEQ ID NO 2 (SB116, in this case named SB2).

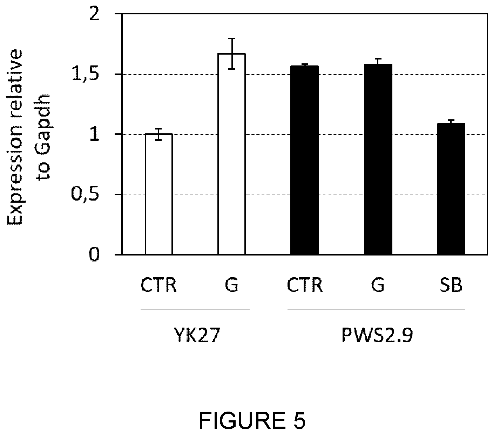

- Figure 5 is a bar chart representing the expression level of DGKK mRNA relative to GAPDH control mRNA in YK27 and PWS2.9 cell lines. YK27 and PWS2.9 cells are human induced pluripotent stem cells generated from an healthy control individual (Martins-Taylor et al. 2011 [8]) and from a PWS patients harboring a microdeletion at 15q11-q13 (Martins-Taylor 2014 [8]), respectively. CTR represents the expression level in cells transfected with control oligonucleotides (G-CTR of SEQ ID NO 10 and SB-CTR of SEQ ID NO 12), G represents the expression level in cells transfected with an oligonucleotide of SEQ ID NO 11 and control oligonucleotide SB-CTR of SEQ ID NO 12, SB represents the expression level in cells transfected with a control oligonucleotide G-CTR of SEQ ID NO 10 and with an oligonucleotide SB116 of SEQ ID NO 2.

### EXAMPLES

### Example 1 : effect of oligonucleotides on Prader-Willi Syndrome and biological mechanism involved

### 1. Material and Methods

### Cell culture

Human cervix carcinoma HeLa S3 cells were grown in Dulbecco's-modified Eagle's medium-high glucose (Sigma-Aldrich) supplemented with 10% fetal bovine serum (Dutscher), 1% penicillin/streptomycin (Sigma-Aldrich) and 1% L-glutamine (Sigma-Aldrich). Human cervix carcinoma HeLa S3 cells were grown in Dulbecco's-modified Eagle's medium-high glucose (Sigma-Aldrich) supplemented with 10% fetal bovine serum (Dutscher), 1% penicillin/streptomycin (Sigma-Aldrich) and 1% L-glutamine (Sigma-Aldrich).

Mouse neuroblastoma Neuro2A cells were grown in Dulbecco's-modified Eagle's medium-high glucose (Sigma-Aldrich) supplemented with 10% fetal bovine serum (Dutscher), 1% penicillin/streptomycin (Sigma-Aldrich) and 1% L-glutamine (Sigma-Aldrich). Human cervix carcinoma HeLa S3 cells were grown in Dulbecco's-modified Eagle's medium-high glucose (Sigma-Aldrich) supplemented with 10% fetal bovine serum (Dutscher), 1% penicillin/streptomycin (Sigma-Aldrich) and 1% L-glutamine (Sigma-Aldrich).

Human induced pluripotent stem cells YK27 and PWS2.9 cells were grown in mTeSR plus medium (STEMCELL) supplemented with mTeSR plus 5X Supplement (STEMCELL). The cells were grown on Vitronectin XF matrix (STEMCELL) using non-tissue culture-treated cultureware.

### Oligonucleotides

The oligonucleotides used in cell transfection were: GGTGGCATTCCTTGGAAAGG (SEQ ID NO 2), GCTCCCTTCAATCCAA (SEQ ID NO 10), TCACTCATTTTGTTCA (SEQ ID NO 11), TAACACGTCTATACGCCCA (SEQ ID NO 12) and AACATTCCTTGGAAAA (SEQ ID NO 13).

Oligonucleotide GGTGGCATTCCTTGGAAAGG (SEQ ID NO 2) is also designated SB-116 or SB2. Oligonucleotide GCTCCCTTCAATCCAA (SEQ ID NO 10) is also designated G-CTR or CTR when used together with SEQ ID NO 12. Oligonucleotide TCACTCATTTTGTTCA (SEQ ID NO 11) is also designated G-116 or G when used together with SEQ ID NO 12. Oligonucleotide TAACACGTCTATACGCCCA (SEQ ID NO 12) is also designated SB-CTR or CTR when used together with SEQ ID NO 10. Oligonucleotide AACATTCCTTGGAAAA (SEQ ID NO 13) is also designated SB1.

### Oligonucleotide Transfection

A total of 200,000 HeLa S3 cells were seeded per well in six-well plates 24 h before transfection by the oligonucleotides. Transfection was performed using Lipofectamine 2000 (Invitrogen) at a final concentration of 16 nM. Samples were collected 24 h post-transfection for total RNA extraction.

A total of 160,000 Neuro 2A cells were seeded per well in six-well plates 24 h before transfection by the oligonucleotides. Transfection was performed using Lipofectamine 2000 (Invitrogen) at a final concentration of 16 nM. Samples were collected 24 h post-transfection for total RNA extraction.

A total of 30,000 YK27 or PWS2.9 cells were seeded per well in six-well plates 72 h before transfection by the oligonucleotides. Transfection was performed using Lipofectamine 3000 (Invitrogen) at a final concentration of 64 nM. Samples were collected 20 h post-transfection for total RNA extraction.

### Total RNA Extraction and RT-qPCR

Total RNAs were collected using the miRNeasy mini kit (QIAGEN) and extracted following the manufacturer's recommendations. The extracted RNAs were quantified using nanodrop 2000. After a DNase step, RNAs were reverse transcribed using the Superscript IV kit (Thermo Fisher Scientific) following the manufacturer's recommendations, using a mix of oligo d(T) and random hexanucleotides. RNA expression level was quantified by real-time quantitative PCR (RT-qPCR) using the iTaq Universal SYBR Green Supermix (Bio-Rad) and StepOne Real-Time PCR system (Applied Biosystems). Quantitative PCR was performed by 40 cycles of the following steps: 15 seconds at 95°C, 15 seconds at 60°C and 15 sec at 72°C followed. After the 40 cycles, a melt curve stage was realized.

The RT-qPCR primers are AACAGCCTCAAGATCATCAGC (SEQ ID NO 4) and GGATGATGTTCTGGAGAGCC (SEQ ID NO 5) for amplification of human Gapdh, TGGATCGATGATGAGTCCC (SEQ ID NO 6) and TGGACCTCAGTTCCGATGAG (SEQ ID NO 7) for amplification of human Snord116, AGAAGCCATCGCTCATCAGT (SEQ ID NO 8) and TGAATTTTCGGAGGAAGACG (SEQ ID NO 9) for amplification of human Dgkk, GGCAAATTCAACGGCACAGT (SEQ ID NO 14) and GGGTCTCGCTCCTGGAAGAT (SEQ ID NO 15) for amplification of mouse Gapdh, GGATCTATGATGATTCCCAG (SEQ ID NO 16) and GGACCTCAGTTCCGATGA (SEQ ID NO 17) for amplification of mouse snord116, GTCACAAGAGCTGCGGTAGT (SEQ ID NO 18) and CTTGGAAAACCGCTTCTGGC (SEQ ID NO 19) for amplification of mouse Dgkk, GCCCGAAAGCCCAACAAGAA (SEQ ID NO 20) and GCTAAGTCCTCGCCCTGTTT (SEQ ID NO 21) for amplification of mouse Nlgn3, ATGCGACAGATCAGCTTGGT (SEQ ID NO 22) and TTCTCCTGGACGTACCCACA (SEQ ID NO 23) for amplification of mouse Rsbn1l, CCGAGCTCCGCAAACTACTA (SEQ ID NO 24) and ATGGCCAAGCGCAGAATCTC (SEQ ID NO 25) for amplification of mouse Nhlh2.

### Identification of snord116 RNA targets

Preliminary work has identified that snord116 function by direct hybridization with target mRNAs Dgkk, Nlgn3, Rsbn1l to alter their expression level and/or splicing level (Baldini 2022 [1]). Evolutionary conservation and theoretical molecular interaction energy data (Baldini et al. 2022 [1]) suggest that the Diacyl glycerol kinase kappa (Dgkk) mRNA is a major target of snord116. Transcriptomic data from PWS mouse models (Zahova 2021 [19]; Knott 2022 [18]) or induced pluripotent cells from PWS patients (Burnett 2017 [17]) support this hypothesis by showing that this mRNA is among the most significantly downregulated.

### Oligonucleotide design

A 20 nucleotide-long sequence of sequence GGXaGGCAXbXcCCXdXeGGAAAGG (SEQ ID NO 1), wherein Xa, Xb, Xc, Xd and Xe are independently T or U was generated. In particular, a 20 nucleotide-long sequence of sequence GGTGGCATTCCTTGGAAAGG (SEQ ID NO 2) was generated and used. These polynucleotides were designed to be complementary to particular RNA sequences, in particular to RNA according to a combination of Watson-Crick and wobble interactions.

### 1. Results

### Use of oligonucleotide of SEQ ID NO 2 and biological/therapeutic effects in PWS model cells.

A transient knockdown of snord116 in Human cervix carcinoma HeLa S3 cell line cells was performed according to the process disclosed in Baldini 2022, to mimic the pathological deregulation state observed in PWS patients and observe its immediate consequences. In particular a Gapmer oligonucleotide directed against snord116 (G-116) of sequence TCACTCATTTTGTTCA (SEQ ID NO 11) was used to transiently knockdown the expression of snord116. A control Gapmer (G-CTR) of sequence GCTCCCTTCAATCCAA (SEQ ID NO 10) has also been used. The extraction and measure of expression level of snord116 RNA and Dgkk mRNA relative to Gapdh control mRNA was carried out according to the above-mentioned process. The obtained Figure 1 represents the results of the steady-state expression level of snord116 and Dgkk mRNAs relative to Gapdh control mRNA. Cells were transfected with the oligonucleotides for 24H before analysis. Black histograms represent the relative expression level in cells transfected with control oligonucleotides, also mentioned as a control Gapmer (G-CTR) of SEQ ID NO 10, white histograms represent the relative expression level in cells transfected with oligonucleotide directed against endogenous snord116 (G-116) of SEQ ID NO 11, generating their knockdown. As shown in Figure 1, the use of a Gapmer oligonucleotide of sequence SEQ ID NO 11 directed against snord116 (G-116) induced a significant decrease in the expression of endogenous snord116 compared to the use of a control Gapmer (G-CTR). As shown in figure 1, the use of a Gapmer oligonucleotide of sequence SEQ ID NO 11 directed against snord116 (G-116) induced a significant increase in the expression of the Dgkk mRNA compared to the use of a control Gapmer (G-CTR).

The extraction and measure of expression level of snord116 and Dgkk mRNAs relative to Gapdh control mRNA in a cell line (Human cervix carcinoma HeLa S3 cells was performed according to the abovementioned process 24H after transfection of the cells with the oligonucleotides of sequence TAACACGTCTATACGCCCA (SEQ ID NO 12) (SB-CTR) or of sequence GGTGGCATTCCTTGGAAAGG (SEQ ID NO 2) also mentioned SB-116. The obtained Figure 2 represents the results of the steady-state expression level of snord116 and Dgkk mRNAs relative to Gapdh control mRNA. Cells were transfected with the oligonucleotides for 24H before analysis. Black histograms represent the relative expression level in cells transfected with control oligonucleotide of SEQ ID NO 12 (SB-CTR), white histograms represent the relative expression level in cells transfected with oligonucleotide (SB-116) of SEQ ID NO 2. As shown in Figure 2, oligonucleotide of SEQ ID NO 2 induces an inverse effect compared to snord116 knockdown on the steady-state expression level of the Dgkk mRNA shown on figure 1. In particular the results clearly demonstrate that oligonucleotide of SEQ ID NO 2 significantly lowers the expression of Dgkk and does not modify the expression of snord 116.

The extraction and measure of expression level of snord116 and Dgkk mRNAs relative to Gapdh control mRNA in the Human cervix carcinoma HeLa S3 cell line was performed according to the above-mentioned process 24H after transfection of the cells with the oligonucleotides of sequence TAACACGTCTATACGCCCA (SEQ ID NO 12) (SB-CTR), of sequence GGTGGCATTCCTTGGAAAGG (SEQ ID NO 2) (SB-116), of sequence GCTCCCTTCAATCCAA (SEQ ID NO 10) (G-CTR) or of sequence TCACTCATTTTGTTCA (SEQ ID NO 11) (G-116). Figure 3 represents the results of steady-state expression level of snord116 and Dgkk mRNAs relative to Gapdh control mRNA obtained. On this figure black histograms represent steady-state expression level in cells transfected with control oligonucleotides, i.e. of SEQ ID NO 10 (G-CTR) and of SEQ ID NO 12 (SB-CTR), white histograms represent steady-state expression level in cells transfected with oligonucleotide of SEQ ID NO 11 (G-116) (directed against endogenous snord116 allowing knockdown of snord 116) and with a control oligonucleotide of SEQ ID NO 12 (SB-CTR), the shaded histograms represent steady-state expression level in cells transfected with oligonucleotide of SEQ ID NO 11 (G-116) and with oligonucleotide of SEQ ID NO 2 (SB116). As shown in Figure 3, the use of the oligonucleotide of SEQ ID NO 2 but not the SB-CTR oligonucleotide restores the steady-state expression level of the Dgkk mRNA upon transient knockdown of endogenous snord116 by the Gapmer oligonucleotide G-116.

The extraction and measure of expression level of Dgkk, Nlgn3, Rsbn1l and Nhlh2 mRNAs relative to Gapdh control mRNA in the Neuro2A mouse neuroblastoma cell line was performed according to the abovementioned process. 24H after transfection of the cells with the oligonucleotides of sequences GCTCCCTTCAATCCAA (SEQ ID NO 10) and TAACACGTCTATACGCCCA (SEQ ID NO 12) (together mentioned CTR on Figure 4) or sequences TCACTCATTTTGTTCA (SEQ ID NO 11) and TAACACGTCTATACGCCCA (SEQ ID NO 12) (together mentioned G on Figure 4), or of sequences TCACTCATTTTGTTCA (SEQ ID NO 11) and AACATTCCTTGGAAAA (SEQ ID NO 13) (together mentioned G+SB1 on Figure 4) or of sequences TCACTCATTTTGTTCA (SEQ ID NO 11) and GGTGGCATTCCTTGGAAAGG (SEQ ID NO 2) (together mentioned G+SB2 on Figure 4). Figure 4 represent the results of steady-state expression level of Dgkk, Nlgn3, Rsbn1L and Nhlh2 mRNAs relative to Gapdh control mRNA obtained. On this figure black histograms represent steady-state expression level in cells transfected with the oligonucleotides of sequence SEQ ID NO 10 and 12 (CTR). Dark histograms represent steady-state expression level in cells transfected with the oligonucleotides of sequence SEQ ID NO 11 and 12 (G). Grey histograms represent steady-state expression level in cells transfected with the oligonucleotides of sequence SEQ ID NO 11 and 13 (G+SB1). Light grey histograms represent steady-state expression level in cells transfected with the oligonucleotides of sequence SEQ ID NO 11 and 2 (G+SB2).

As shown in Figure 4, the use of the oligonucleotide of SEQ ID NO 2 (SB2) but not the oligonucleotide of SEQ ID NO 13 (SB1) restores the steady-state expression level of the Dgkk, Nlgn3, Rsbn1L and Nhlh2 mRNA upon transient knockdown of endogenous snord116 by the oligonucleotide of sequence SEQ ID NO 11 (G). In particular, the results clearly demonstrate that oligonucleotide of SEQ ID NO 13 (SB1) induces, when the Snord 116 gene is knock down, an overly reduction of expression of Dgkk and Rsbn1l, no significant modification of expression of Nlgn3, and an over expression of Nhlh2. To the contrary, the results clearly demonstrate that oligonucleotide of SEQ ID NO 2 (SB2) induces, when the Snord 116 gene is knock down, a wild-type expression of Dgkk, Nlgn3, Rsbn1l and Nhlh2, in other words Oligonucleotide GGTGGCATTCCTTGGAAAGG (SEQ ID NO 2) enables effective correction of expression of all four genes.

The extraction and measure of expression level of Dgkk mRNA relative to Gapdh control mRNA in two human induced pluripotent stem cell lines YK27 and PWS2.9 was performed according to the above-mentioned process 20H after transfection of the cells with the oligonucleotides of sequence TAACACGTCTATACGCCCA (SEQ ID NO 12) (SB-CTR) or of sequence GGTGGCATTCCTTGGAAAGG (SEQ ID NO 2) (SB-116). The obtained Figure 5 represents the results of the steady-state expression level of Dgkk mRNA relative to Gapdh control mRNA. Cells were transfected with the oligonucleotides for 20H before analysis. White histograms represent the relative expression level in cells transfected with control oligonucleotides of SEQ ID NO 10 and 12 (Mentioned CTR on figure 5) and oligonucleotides of SEQ ID NO 11 and 12 (Mentioned G on figure 5) in the human YK27 cell line issued from a healthy donor, black histograms represent the relative expression level in cells transfected with control oligonucleotides of SEQ ID NO 10 and 12 (CTR), oligonucleotides of SEQ ID NO 11 and 12 (G) or oligonucleotides of SEQ ID NO 10 and 2 (Mentioned SB on figure 5), in the human PWS2.9 cell line issued from a PWS patient donor. As shown in Figure 5, in the PWS cell line the oligonucleotide of SEQ ID NO 2 reverses the steady-state expression level of Dgkk to a normal level as observed in the healthy cell line. In particular the results clearly demonstrate that oligonucleotide of SEQ ID NO 2 significantly lowers the expression of Dgkk in a cell line model of PWS issued from a patient.

This example clearly demonstrates that the present invention allows surprisingly to restore the expression level of gene responsible of PWS, for example Dgkk gene, in cell line model of PWS. In other words, this example clearly demonstrate that the present invention allows in cell line model of PWS to regulate the expression of genes involved in PWS, in particular to have a normal expression level of Dgkk allowing therefore the treatment of PWS and/or Prader Willy-like syndrome.

This example clearly demonstrates that the present invention provides and an efficient treatment and/or effective treatment of Prader Willi syndrome (PWS) and/or Prader Willy-like syndrome.

### References

1. Baldini L, Robert A, Charpentier B, Labialle S. Phylogenetic and Molecular Analyses Identify SNORD116 Targets Involved in the Prader-Willi Syndrome. Mol Biol Evol. 2022. 39(1):msab348.
2. Corey DR. Nusinersen, an antisense oligonucleotide drug for spinal muscular atrophy. Nat Neurosci. 2017. 20(4):497-499.
3. Cruvinel E, Budinetz T, Germain N, Chamberlain S, Lalande M, Martins-Taylor K. Reactivation of maternal SNORD116 cluster via SETDB1 knockdown in Prader-Willi syndrome iPSCs. Hum Mol Genet. 2014. 23(17):4674-85.
4. de Smith AJ, Purmann C, Walters RG, Ellis RJ, Holder SE, Van Haelst MM, Brady AF, Fairbrother UL, Dattani M, Keogh JM, Henning E, Yeo GS, O'Rahilly S, Froguel P, Farooqi IS, Blakemore AI. A deletion of the HBII-85 class of small nucleolar RNAs (snoRNAs) is associated with hyperphagia, obesity and hypogonadism. Hum Mol Genet. 2009. 18(17):3257-65.
5. Duker AL, Ballif BC, Bawle EV, Person RE, Mahadevan S, Alliman S, Thompson R, Traylor R, Bejjani BA, Shaffer LG, Rosenfeld JA, Lamb AN, Sahoo T.Paternally inherited microdeletion at 15q11.2 confirms a significant role for the SNORD116 C/D box snoRNA cluster in Prader-Willi syndrome. Eur J Hum Genet. 2010. 18(11):1196-201.
6. Finkel RS, Chiriboga CA, Vajsar J, Day JW, Montes J, De Vivo DC, Yamashita M, Rigo F, Hung G, Schneider E, Norris DA, Xia S, Bennett CF, Bishop KM. Treatment of infantile-onset spinal muscular atrophy with nusinersen: a phase 2, open-label, dose-escalation study. Lancet. 2016 Dec 17;388(10063):3017-3026.
7. Juriaans AF, Kerkhof GF, Hokken-Koelega ACS. The Spectrum of the Prader-Willi-like Pheno- and Genotype: A Review of the Literature. Endocr Rev. 2022 Jan 12;43(1):1-18.
8. Martins-Taylor K, Nisler BS, Taapken SM, Compton T, Crandall L, Montgomery KD, Lalande M, Xu RH. Recurrent copy number variations in human induced pluripotent stem cells. Nat Biotechnol. 2011 Jun 7;29(6):488-91.
9. Martins-Taylor K, Hsiao JS, Chen PF, Glatt-Deeley H, De Smith AJ, Blakemore AI, Lalande M, Chamberlain SJ. Imprinted expression of UBE3A in non-neuronal cells from a Prader-Willi syndrome patient with an atypical deletion. Hum Mol Genet. 2014 May 1;23(9):2364-73.
10. Kim Y, Lee HM, Xiong Y, Sciaky N, Hulbert SW, Cao X, Everitt JI, Jin J, Roth BL, Jiang YH. Targeting the histone methyltransferase G9a activates imprinted genes and improves survival of a mouse model of Prader-Willi syndrome. Nat Med. 2017. 23(2):213-222.
11. Roberts TC, Langer R, Wood MJA. Advances in oligonucleotide drug delivery. Nat Rev Drug Discov. 2020. 19(10):673-694.
12. Rocha CF, Paiva CL. Prader-Willi-like phenotypes: a systematic review of their chromosomal abnormalities. Genet Mol Res. 2014 Mar 31;13(1):2290-8.
13. Sahoo T, del Gaudio D, German JR, Shinawi M, Peters SU, Person RE, Garnica A, Cheung SW, Beaudet AL. Prader-Willi phenotype caused by paternal deficiency for the HBII-85 C/D box small nucleolar RNA cluster. Nat Genet. 2008. 40(6):719-21.
14. Sheng L, Rigo F, Bennett CF, Krainer AR, Hua Y. Comparison of the efficacy of MOE and PMO modifications of systemic antisense oligonucleotides in a severe SMA mouse model. Nucleic Acids Res. 2020. 48(6):2853-2865.
15. Tabet R, Moutin E, Becker JA, Heintz D, Fouillen L, Flatter E, Kr 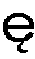 żel W, Alunni V, Koebel P, Dembélé D, Tassone F, Bardoni B, Mandel JL, Vitale N, Muller D, Le Merrer J, Moine H. Fragile X Mental Retardation Protein (FMRP) controls diacylglycerol kinase activity in neurons. Proc Natl Acad Sci U S A. 2016. 113(26):E3619-28.
16. Tan Q, Potter KJ, Burnett LC, Orsso CE, Inman M, Ryman DC, Haqq AM. Prader-Willi-Like Phenotype Caused by an Atypical 15q11.2 Microdeletion. Genes (Basel). 2020. 11(2):128.
17. Burnett LC, LeDuc CA, Sulsona CR, Paull D, Rausch R, Eddiry S, Carli JF, Morabito MV, Skowronski AA, Hubner G, Zimmer M, Wang L, Day R, Levy B, Fennoy I, Dubern B, Poitou C, Clement K, Butler MG, Rosenbaum M, Salles JP, Tauber M, Driscoll DJ, Egli D, Leibel RL. Deficiency in prohormone convertase PC1 impairs prohormone processing in Prader-Willi syndrome. J Clin Invest. 2017. 127(1):293-305.
18. Knott B, Kocher MA, Paz HA, Hamm SE, Fink W, Mason J, Grange RW, Wankhade UD, Good DJ. Dietary Conjugated Linoleic Acid Reduces Body Weight and Fat in Snord116m+/p- and Snord116m-/p-Mouse Models of Prader-Willi Syndrome. Nutrients. 2022. 14(4):860.
19.Zahova SK, Humby T, Davies JR, Morgan JE, Isles AR. Comparison of mouse models reveals a molecular distinction between psychotic illness in PWS and schizophrenia. Transl Psychiatry. 2021. 11(1):433.
20.Sproat et al., (1991) Nucle. Acid. Res. 19, 733-738;
21. Cotton et al., (1991) Nucl. Acid. Res. 19, 2629-2635;
22. Hobbs et al., (1973) Biochemistry 12, 5138-5145.
23. Smith and Waterman, Adv. Appl. Math., 2:482 (1981) https://doi.org/10.1016/0196-8858(81)90046-4
24. Needleman SB, Wunsch CD. A general method applicable to the search for similarities in the amino acid sequence of two proteins. J Mol Biol. 1970 Mar;48(3):443-53. doi: 10.1016/0022-2836(70)90057-4. PMID: 5420325.
25. Pearson WR, Lipman DJ. Improved tools for biological sequence comparison. Proc Natl Acad Sci USA. 1988 Apr;85(8):2444-8. doi: 10.1073/pnas.85.8.2444. PMID: 3162770; PMCID: PMC280013.
26. Murray E. J. (ed.), Methods in Molecular Biology, Vol. 7, Gene Transfer and Expression Protocols, Humana Press (1991)
27.Johnston, S. Biolistic transformation: microbes to mice. Nature 346, 776-777 (1990). https://doi.org/10.1038/346776a0
28. Brash et al., Mol. Cell Biol., 7: 2031-2034 https://doi.org/10.1128/mcb.7.5.2031-2034.1987
29. Sproat et al., (1991) Nucle. Acid. Res. 19, 733-738
30. Matthew Cotten et al., 2'-O-methyl, 2'-O-ethyl oligoribonucleotides and phosphorothioate oligodeoxyribonucleotides as inhibitors of the in vitro U7 snRNP-dependent mRNA processing event, Nucleic Acids Research, Volume 19, Issue 10, 11 May 1991, Pages 2629-2635, https://doi.org/10.1093/nar/19.10.2629
31. John Hobbs, Hans Sternbach, Mathias Sprinzl, and Fritz Eckstein, , Polynucleotides containing 2'-amino-2'-deoxyribose and 2'-azido-2'-deoxyribose, Biochemistry 1973 12 (25), 5138-5145, DOI: 10.1021/bi00749a018
32. US 5 660 985
33. US 5 756 703

## Claims

1. An isolated polynucleotide comprising the sequence GGXaGGCAXbXcCCXdXeGGAAAGG (SEQ ID NO 1), wherein Xa, Xb, Xc, Xd and Xe are independently T or U.

2. An isolated polynucleotide according to claim 1 wherein the isolated polynucleotide has at least 65% sequence identity with sequence SEQ ID NO 1.

3. An isolated polynucleotide according to claim 1 wherein the isolated polynucleotide has at least 65% sequence identity with sequence GGTGGCATTCCTTGGAAAGG (SEQ ID NO 2).

4. An isolated polynucleotide according to claim 1 wherein the isolated polynucleotide has at least 65% sequence identity with sequence GGUGGCAUUCCUUGGAAAGG (SEQ ID NO 3).

5. An isolated polynucleotide according to claim 1 wherein the isolated polynucleotide is of sequence GGTGGCATTCCTTGGAAAGG (SEQ ID NO 2) or GGUGGCAUUCCUUGGAAAGG (SEQ ID NO 3).

6. An isolated polynucleotide according to claim 1 wherein the isolated polynucleotide is of sequence GGTGGCATTCCTTGGAAAGG (SEQ ID NO 2).

7. An isolated polynucleotide according to anyone of the preceding claims, comprising at least one modification chosen among biostability-enhancing chemical modifications such as locked nucleic acids and/or at least one modified sugar residue.

8. A pharmaceutical composition comprising isolated polynucleotide as defined in claims 1 to 7 and a pharmaceutical acceptable support.

9. An isolated polynucleotide according to anyone of claim 1 to 7 for use in the treatment of Prader-Willi Syndrome and/or Prader Willi-like syndromes.
